# EUROPEAN PATENT APPLICATION

(11) **EP 2 989 903 A1**
(43) Date of publication of application: **02.03.2016**
(21) Application number: 14776366.8
(22) Date of filing: 27.03.2014
(51) Int. Cl.: A23L 33/00, A23L 33/10, A23L 27/30, A23L 33/17, A23L 33/21, A23L 33/15, A23L 33/16, A61K 31/7016, A61P 25/00

(54) **FOOD SUPPLEMENT FOR PEOPLE WITH DOWN SYNDROME, AUTISM SPECTRUM DISORDER AND/OR ATTENTION DEFICIT DISORDER WITH OR WITHOUT HYPERACTIVITY**

(30) Priority: 27.03.2013 MX 2013003636
(71) Applicant: PALSGAARD INDUSTRI DE MEXICO, S. DE R.L.DE C.V., 78433 San Luis Potosi, S.L.P. (MX)
(72) Inventor: SÁNCHEZ, Edilberto, 78433 San Luis Potosi, S.L.P. (MX)
(74) Representative: Wibbelmann, Jobst
(86) International application number: PCT/IB2014/000437
(87) International publication number: WO 2014/155186

(57) **Abstract**

The present invention describes a food supplement comprising isomaltulose, milk serum protein concentrate, linseed, milk serum mineral complex, short chain fructooligosacharides, lactoferrin, and vitamin B6.

This food supplement was developed for use as support in the nutritional therapy in people suffering from Trisomy 21, Autism Spectrum Disorders and/or Attention Deficit Disorder with or without Hyperactivity.

## Description

### FIELD OF THE INVENTION

The present invention is related to the Food Industry, specifically to human beings nutrition, and more particularly it is related to a food supplement for people with Trisomy 21, Autism Spectrum Disorders and/or Attention Deficit Disorder with or without Hyperactivity.

### BACKGROUND OF THE INVENTION

According to data corresponding to 2009, around 37 thousand people comprise the population with some condition in the Autism Spectrum Disorders (ASD) in Mexico ("La Jornada en la ciencia" Journal of February 20, 2009: "There are 37 thousand autistic children in Mexico" Flores, Javier). With respect to Trisomy 21 or Down Syndrome, it is possible to estimate that in Mexico the amount of people with this condition is around 150 thousand individuals, according to the information published by the Health Ministry of the Mexican government in the document "Technical Guidelines for the Integral Attention for People with Down Syndrome", with respect to the birth rate of people with said condition; the number of births reported by the Population and Housing census carried out by the National Institute of Statistics and Geography (INEGI) from 1930 to 2010; and, the average life expectancy data for the people with Trisomy 21 shown at the press conference offered by the Health Secretary in 2006.

On the other hand, in the report of the "Specific Program for Attention Deficit Disorder of the SSA 2001-2006", mention is made of a prevalence of 1 million 500 thousand people at children age and teenagers with this disorder, figure that might double considering that adults still suffer from the disorder (Health Ministry, Mexican Government, 2006).

While people groups within ASD and Down Syndrome are still considered in our country as vulnerable minorities, the increasing percentage of the population affected with Attention Deficit Disorder with Hyperactivity (ADHD) or without Hyperactivity (ADD), makes them a public health focus of attention.

Based on the numbers from the Population and Housing Census carried out in 2010 by the National Institute of Statistics and Geography (INEGI), there are 5 million 739 thousand 270 people in Mexico with some kind of physical or mental disability. Considering the data shown in previous paragraphs, at least 30% of the disabled population in Mexico falls in the ASD, Trisomy 21, ADD or ADHD condition.

The etiology of the affections within the ASD, Trisomy 21, ADD and ADHD, has not been clearly determined yet, and therefore the therapeutic management developed to date is focused on ameliorating the symptoms decreasing the quality of life and hindering cognitive processes and, consequently, social participation of people suffering thereof. Nevertheless, multiple affinities of the physiological, metabolic, immune, and genetic type, as well as in therapeutic management of people diagnosed with these disorders have been reported in the literature, whereby diverse studies for the therapeutic treatment thereof have been made through pharmacological dosing models, which effectiveness has shown to be limited and even ineffective in many cases, and diminished if not accompanied by behavioral-type therapies.

For example, stimulants such as methylphenidate and dextroamphetamines are some of the most common treatments supplied to people with Trisomy 21, ASD and/or ADD (Rappley, 2005; Frohlich et al., 2007; Findling, 2008). These drugs have a direct incidence in neurotransmitters performance, such as norepinephrine and dopamine, intimately related to cognition disposition and to anxiety and satiety sensations. Some of the positive effects related to the supply of these compounds are associated to reduced impulsiveness and hyperactivity, as well as higher focusing ability; however, in spite of their effectiveness in such aspects, they are also accompanied by adverse effects such as propensity to develop an intake dependence (addictions), body weight imbalance, as well as severe appetite affections (anorexia or obesity), growth issues (size), severe gastrointestinal tract problems, migraine, severe sleep disorders, considerable variations in blood pressure (Rappley, 2005; Frohlich et al, 2007; Findling, 2008; Najib, 2008), and considerable hormonal imbalances. Possible side effects from the consumption of said drugs make both the consumers and their legal representatives (parents or tutors) desist in their use, choosing behavioral-type multidisciplinary therapies, which did not yield convincing results either.

This has lead researchers and patients' relatives to seek for alternative treatments for such disorders, focusing on the nutritional variables as one of the definitive and fundamental factors for combating, operation and displaying of these affections.

According to the above, diverse food supplements have been developed which focus of interest is to meet the needs of these populations of people:

The first one of them was developed in the 1940s in the United States by Dr. Henry Turkel, who administered a mixture of 48 ingredients including vitamins, minerals and hormones to children with Trisomy 21 (Turkel, 1975). Although Dr. Turkel's mixture was administered for almost forty years to children with Trisomy 21, up to date there has not been any report of these people having been benefited with the product.

Another one was developed in the 1960s by Dr. Habound in Germany (Habound, 1955), which consisted of a mixture of vitamins, hormones and enzymes. In the case of the supplement developed by Dr. Habound, the benefits to the administered subjects were also non-proven.

Likewise, in the 1980s, Dr. Ruth Harrell together with a researchers team developed a vitamin, mineral and thyroid hormone based supplement whose preliminary studies suggested improvements in the Intelligence Quotient scorings (IQ) of the administered people, by causing "physical changes towards normality" (Harrell, 1981), obtaining prominent results in three people with Down Syndrome at the time of the research. Nevertheless, more recent studies on this supplement have not replicated the positive results reported.

Likewise, a product based on Dr. Turkel's original mixture also reached the market in the 80s. This product is still administered together with thyroid hormone to people with Down Syndrome, although scientific studies have not been published showing the effectiveness thereof.

In the 1990s a product appeared with a considerably higher market penetration than the previous works, since it was promoted in a television show; the product was created by Dr. Dixie Lawrence Tafoya on the basis of Dr. Turkel's original mixture, but adding more ingredients. This formula was originally tested on Dr. Lawrence's own adoptive daughter with Down Syndrome, and although people in her school environment appreciated improvements in her performance, the U.S. National Down Syndrome Congress could not support the results since the girl was also being treated with neurological drugs (Piracetam).

Further, a similar formula to that of the above product also developed by Dr. Lawrence was commercialized with a different dose. This new product contained about 40 ingredients among vitamins and minerals, and currently it is still marketed with some modifications, such as the addition of antioxidants and the removal of some ingredients for being possible allergen agents. The company commercializing the product states the positive effects the nutrients may have on people with Down Syndrome, but it gives no documentary evidence of specific cases of people having had significant changes from their regular intake.

On the other hand, with respect to food supplements for people with ASD and/or ADD, there is a vitamin, mineral and amino acid complex to help detoxifying and calming down children with autism, which was developed and marketed in Singapore by the "Autism Recovery Centre". However, as with the above products, results of a scientific study are not offered showing the effectiveness of this supplement in people being under treatment.

Further, a product with the name Neurizen is commercialized to optimize memory, attention, concentration and brain lucidity, which supplements and enhances the contribution of amino acids, vitamins and essential minerals for a better cerebral chemistry function and it can be employed in people with ADD. This low glycemic index formulation contains milk serum protein and soy protein, as well as dietetic fiber, vitamins (including vitamin B6) and minerals; however, there is not documentary evidence supporting the changes stated to be perceived in the people consuming it.

On the other hand, U.S. Patent No. 5,902,797 describes a food supplement directed to children suffering from ADD or ADHD, especially to those whose appetite is being affected by a psychostimulant medication treatment. This supplement comprises carbohydrates, proteins and fats among its components, optionally also containing vitamins, minerals, fibers, antioxidants, etc.

International Publication No. WO 02/43507 discloses food supplements comprising: a) minerals capable of promoting metallothioneins (zinc and selenium); b) vitamins capable of promoting metallothioneins (vitamin B6, A, C, E, and coenzyme-shaped vitamin B6); c) non-essential amino acids (taurine) effective to remove excess of metals in the bloodstream; and, d) agents capable of an *in vivo* cysteine release (glutathione). It also may contain, optionally, an amino acid mixture effective to promote metallothioneins in a subject (serine, lysine, alanine, glycine, threonine, etc.). Optionally, flavoring agents may be included in order to render the formulations more pleasant. These food supplements are preferably used to treat autism, although they may also turn out to be useful in the treatment of other diseases such as ADHD.

Finally, International Publication No. WO 2008/071991 discloses a nutritional formulation for treating pervasive developmental disorders (such as autism). This formulation contains free amino acids as the only protein source present in the formulation, as well as a fat source comprising long chain polyinsaturated fatty acids, and/or dietetic fibers, and/or higher levels of micronutrients. As dietetic fibers, trans-galactooligosacharides, long chain fructooligosacharides (FOS), short chain FOS, etc. may be used. Likewise, as micronutrients, vitamin B6 and minerals such as magnesium, zinc and calcium, among others, may be used.

As can be seen, it is possible to find diverse nutritional supplements in the prior art which claim to help treating people with Syndrome Down, Autism Spectrum Disorders or Attention Deficit Disorder with or without Hyperactivity; however, there is no convincing evidence showing they actually do; therefore, there is a need for a nutritional supplement specifically developed to support the nutritional therapy of people with these disorders in a really effective manner, and which components act in such a way that they complement the metabolic pathways of these people.

### OBJECTS OF THE INVENTION

Considering the drawbacks in the prior art, it is an object of the present invention to provide a food supplement for people with Trisomy 21, Autism Spectrum Disorders and/or Attention Deficit Disorder with or without Hyperactivity, specifically developed to support the nutritional therapy of people suffering from said disorders.

A further object of the present invention is to provide a food supplement for people with Trisomy 21, Autism Spectrum Disorders and/or Attention Deficit Disorder with or without Hyperactivity, to help regulate altered metabolic processes and reducing feed sensitivities in people suffering from said disorders.

It is another object of the present invention to provide a food supplement for people with Trisomy 21, Autism Spectrum Disorders and/or Attention Deficit Disorder with or without Hyperactivity, to help them reinforce their immune system and allow them to make better use of proteins, calcium, phosphorous and iron.

A further object of the present invention is to provide a food supplement for people with Trisomy 21, Autism Spectrum Disorders and/or Attention Deficit Disorder with or without Hyperactivity, allowing them to have a more efficient digestive process, reducing intestinal hypotonia, inflammation and chronic intestinal irritation.

An additional object of the present invention is to provide a food supplement for people with Trisomy 21, Autism Spectrum Disorders and/or Attention Deficit Disorder with or without Hyperactivity, allowing them to have higher neurotransmitters production and a better utilization thereof, in order to reach a greater focusing and attention capability, as well as to have improvements in their behavior and socially acceptable interactions.

### SUMMARY OF THE INVENTION

To that end, a food supplement has been invented characterized by comprising: a) from 62 to 75% isomaltulose; b) from 12 to 21% milk serum protein concentrate; c) from 2 to 8.4% linseed; d) from 1 to 4.4% milk serum mineral complex; and) from 1.6 to 6.7% short chain fructooligosacharides; f) from 0.03 to 0.09% lactoferrin; and, g) from 0.002 to 0.008% vitamin B6.

Another aspect of the invention considers the use of this food supplement as a support in the nutritional therapy for people suffering from Trisomy 21, Autism Spectrum Disorders or Attention Deficit Disorder with or without Hyperactivity.

### BRIEF DESCRIPTION OF THE DRAWINGS

The novel featuring aspects of the present invention will be set forth in particular in the appended claims. Nevertheless, some embodiments, features and some objects and advantages thereof, will be better understood in the detailed description, when read together with the appended drawings, wherein:
Fig. 1 is a chart showing the relationship between average values of the total time the subject remained in bed (TTC), total time the recording lasted (TTR), and the total sleep time (TTS) in populations with T21, TEA y TDA.
Fig. 2 is a chart in which the distribution of the four stages of sleep during the TTS in the populations under study is observed.
Fig. 3 shows the number of sleep apneas per hour in populations with T21, ASD and ADD.

### DETAILED DESCRIPTION OF THE INVENTION

It has been surprisingly found that the food supplement of the present invention has a synergic effect allowing to efficiently support the nutritional therapy of people suffering from Autism Spectrum Disorders (ASD), Trisomy 21 or Attention Deficit Disorder with or without Hyperactivity, (ADHD) or (ADD) respectively, helping to regulate the altered metabolic processes and reduce the feed sensitivities in these people.

Therefore, in one aspect of the present invention, a food supplement is described, comprising: a) from 62 to 75% isomaltulose; b) from 12 to 21% milk serum protein concentrate; c) from 2 to 8.4% linseed; d) from 1 to 4.4% milk serum mineral complex; and) from 1.6 to 6.7% short chain fructooligosacharides; f) from 0.03 to 0.09% lactoferrin; and, g) from 0.002 to 0.008% vitamin B6.

Optionally, the food supplement of the present invention may comprise a flavoring agent, preferably in an amount from 0.02 to 10% by weight.

In a specific embodiment of the present invention, the food supplement comprises: a) from 65 to 71% isomaltulose; b) from 14 to 17% milk serum protein concentrate; c) from 4 to 4.5% linseed; d) from 2 to 2.5% milk serum mineral complex; and) from 3 to 4% short chain fructooligosacharides; f) from 0.05 to 0.07% lactoferrin; and, g) from 0.005 to 0.007% vitamin B6.

Optionally, the food supplement of the present invention may comprise a flavoring agent, preferably in an amount from 0.02 to 10% by weight.

For the purpose of the present invention, isomaltulose (6-0-α-D-glucopyranosyl-D-fructofuranose), is a disaccharide consisting of a glucose unit and a fructose unit linked together through a glycocidic bond α-1,6. Preferably, isomaltulose is obtained from beet sucrose by means of an enzymatic rearrangement leading to a change in the glycocidic bond of a α-1,2 structure in the sucrose, to a α-1,6 structure in isomaltulose.

Isomaltulose is a low glycemic index sweetener and it is a powerful source of constant energy for an extended period of time, unlike sucrose, which energetic curve has a very high peak of caloric contribution, being quickly lost. Furthermore, it is a non-fermentable carbohydrate, and therefore it is a non-proliferation and overproduction substrate for pathogenic agents such as Candida, of which subjects of populations with ASD, Trisomy 21 and ADD or ADHD are easy prey. Likewise, the isomaltulose has the same osmotic pressure as sucrose, then, when in solution with other nutrients, promotes their absorption by the body with greater efficacy.

With respect to the milk serum protein concentrate, this has a protein content of about 80%; being preferably obtained by an ultrafiltration process and it has an amino acid profile similar to breast milk including all essential amino acids, thus having a protein efficiency ratio (PER) o bioavailability from 3.2 and 3.9, i.e., higher than any other protein.

Referring to linseed, this is preferably crushed and it has the following composition: about 41% fat, about 28% dietetic fiber, about 20% plant protein, about 7% humidity and about 4% ashes. In addition, it has two parts of omega-6 fatty acids to three parts of omega-3 fatty acids ratio. To achieve this composition, the linseed grains are selected in their optimum ripening time and they are subjected to a specialized grinding process preserving the fatty cell micelles intact, meaning that the contained polyinsaturated essential fatty acids reach the body in the best nutritional shape. In a particularly preferred embodiment of the invention, the linseed employed in the composition is golden linseed.

Preferably, the milk serum mineral complex is obtained by subjecting the milk serum to an ultrafiltration process, and having the following composition: about 24% calcium, about 12.50% phosphorous, about 1.50% magnesium, about 0.80% potassium, about 0.0080% zinc and about 0.0004% copper, thereby its composition is almost the same as that of human bones. The milk serum mineral complex has a pH of less than 4.5, which makes it completely soluble when arriving at the stomach.

Referring to the short chain fructooligosacharides (FOS), they are preferably produced from beet-sucrose oligomers, through the action of the endocellular enzyme β-fructofuranosidase from a genetically non-modified *Aspergillus japonicus* Saito strain (ATCC No. 20611). The short chain FOS have the formula 1F-(1-β-fructofuranosyl)ₙ₋₁, where "n" may vary between 2 and 4.

On the other hand, the lactoferrin being used is extracted from milk serum through a patented process to preserve its best bioactivity state. Lactoferrin can be found in a hololactoferrin (iron rich) or apolactoferrin (iron-free) form.

Referring to the vitamin B6, this may be used in the pyridoxine or pyridoxine hydrochloride form, the pyridoxine hydrochloride being preferred.

Optionally, one or more flavoring agents may be used, natural or artificial, preferably natural, in order to improve palatability of the food supplement. The flavoring agent concentration will depend on the used flavor and may be, for example, up to 13% by weight. Likewise, the food supplement may comprise from 2.5 to 5% by weight of a sweetener, preferably a stevia-based sweetener.

Moreover, in order to achieve a better bioavailability of the components conforming the food supplement of the present invention, it is preferred that the osmolarity thereof be in the range of 170 to 295 mOsm/L.

The nutritional supplement of the present invention is preferably administered orally and it may be in the following forms: powder for reconstituting, tablet, lozenge, pill, capsule, chewable unit dose, sachet, suspension, lollipop, candy, rubbery or syrup, preferably in a powder form to reconstitute in a liquid.

In a preferred embodiment, the food supplement of the present invention is administered twice a day; the first intake is performed in the morning before the first meal, while the second intake is performed in the afternoon. More preferably, the first intake is performed before the first meal of the day, while the second intake is carried out about 2 hours before night sleep.

As mentioned above, the present nutritional supplement is used in people suffering from Trisomy 21, ASD and/or ADD or ADHD, allowing to support the nutritional therapy of these people in an efficient manner, since it helps to regulate altered metabolic processes, reduce feed sensitivities in these people, and reinforce the immune system, allowing for a better use of proteins, calcium, phosphorous and iron, achieving a more efficient digestive process, reducing intestinal hypotonia, chronic intestinal inflammation and irritation; the above as a whole, achieves a higher concentration and attention ability, as well as an improvement in socially acceptable behavior, in people with ASD, Trisomy 21 and/or ADD or ADHD.

It is worth pointing out that although the food supplement of the present invention allows to support the nutritional therapy of people suffering from Autism Spectrum Disorders (ASD), Trisomy 21 or Attention Deficit Disorder with or without Hyperactivity, (ADHD) or (ADD) in an efficient manner, it may also be used in people without affections or neurotypical, in which it will also have positive effects.

In a further embodiment of the invention, the food supplement may be used in combination with a diet wherein milk proteins, gluten, high glycemic index sugar, and red meat proteins have been replaced by casein-free milk, low glycemic index sugar, gluten-free flour products, and white meat or plant proteins.

As mentioned above, the food supplement of the present invention has a synergistic effect allowing for the support of the nutritional therapy of people suffering from ASD, Trisomy 21 and/or ADD or ADHD in an efficient manner. This is because said supplement intervenes in various metabolic pathways and biochemical processes of the body, not only because of the composing elements, but because their interaction allows them to contribute to the nutritional balance of a yet broader spectrum of systems and body organs, which would not occur if the ingredients were supplied separately.

The interaction between the components starts when the subject takes the supplement. Since the isomaltulose is a low glycemic index carbohydrate, it has a very low impact in the production of insulin; however, it gives constant glucose levels to the locomotive apparatus and nervous system, which translates in a limited but balanced and longer energy flow, (compared to sucrose or glucose), thereby making the body use the fat available in food and in the body reserve deposits. Likewise, isomaltulose is a non-bacterial enzymatic hydrolisis carbohydrate, therefore it is not a suitable substrate for the pathogenic microorganism proliferation, particularly Candida; thereby preventing said pathogens from colonizing the various organs in the digestive apparatus and, consequently, interrupting the biochemical processes and impeding nutrient absorption essential to preserve a healthy quality life.

In addition to the above isomaltulose effect, lactoferrin acts as a fungicide, antibacterial and antiviral agent, making it an important ally to the immune system. Added to the isomaltulose and lactoferrin effect, the short chain FOS constitute a precursor of the beneficial bacteria (prebiotics) living in the bowel, contributing to better nutrients absorption. Likewise, the dietetic fiber contribution offered by the golden linseed favors intestinal motility, resulting in a reduced traffic time of fecal bolus through the tract, and coadjuvating to protect the balance of the intestinal flora, and also maintain proper absorption levels of the essential nutrients in this part of the body.

On the other hand, the synergy occurring between isomaltulose and golden linseed is due to, as mentioned above, since isomaltulose is a slow absorption carbohydrate, the body is forced to consume fat, thereby the body consumes fat to balance the energetic loss; this added to the high bioavailability of the golden linseed, renders a more efficient metabolism of the omega-3, omega-6 and omega-9 essential fatty acids; reflected on a suitable neurotransmitters production level. More specifically, the metabolic sequence of the fatty acids in the body is inversely proportional to the constitution of the golden linseed used in the supplement of the present invention, i.e., the body starts the cleavage of the oleic acid (omega-9), continues with linolenic acid (omega-6) and finally works on the alpha-linoleic acid (omega-3), the latter being one of the main elements related to neurotransmitters production. In order for the body to be able to carry out said metabolic processes, it requires catalysts to transform fatty acids from their basic forms to the compounds playing an essential role in the production and distribution of neurotransmitters; in this way, the milk serum mineral complex interacts with the golden linseed, contributing with magnesium and zinc, which together with vitamin B6 transform the omega-9 acid and the omega-6 acid in araquidonic acid, and the omega-3 acid in eicosapentaenoic acid and docosahexaenoic acid. The metabolic pathway of these acids continues with prostaglandins production, exerting vital effects in anti-inflammatory and inflammatory processes, as well as in the blood stream reaching the brain.

It is known that one of the protein functions is restoring and maintaining body tissues in optimum conditions, then the milk serum protein concentrate contributes to restore integrity of the bowel walls from injuries caused by pathogenic microorganisms, empowering the synergy occurred between isomaltulose, lactoferrin and short chain fructooligosacharides. Likewise, it has been reported that about 95% of serotonin and 50% of dopamine bioavailable and used in the body are produced in the small intestine (Manocha et al., 2012; Gershon, 1999; Mirre, 2012); then, it is very important to restore and to protect the integrity of said organ to guarantee a suitable production and utilization of the main neurotransmitters regulating mental and physical activities.

Now then, it is known that short chain FOS intake favors a better calcium bone-assimilation; this effect is empowered by the contribution of the best calcium contained in the milk serum mineral complex; thereby it is possible to avoid the reduction of said bone mineral to divert it to other biochemical processes and thus reducing calcium deficiency and possible bone fractures. Similarly, it is essential to have vitamin K contribution for bone calcium fixation and its assimilation in the process of blood coagulation and neuronal synapse. However, in human beings this vitamin is produced via the intestinal flora and, therefore, in disbiotic and/or injured organisms, whichever the cause, such as damage to the intestine walls caused by pathogenic microorganisms, its production is affected (Berkner and Runge, 2004). From the above, the incorporation of golden linseed to the nutritional supplement formulation of the present invention is essential, among other reasons, because it is a natural source of vitamin K, achieving a calcium maximum assimilation in the body.

The present invention will be better understood from the following examples, which are shown for illustrative purposes only to allow a proper understanding of the preferred embodiments of the present invention, without implying that there are no other embodiments non-illustrated which may be practiced based on the above disclosed detailed description.

### EXAMPLES

### Example 1. Evaluation of the food supplement in people with Trisomy 21

A year-period analytic study was carried out in people with Trisomy 21 or Down Syndrome. The study population was comprised of 34 subjects between 2 and 52 years old attending the Centro Estancia y Habilitación Fundación CTDUCA Atención Integral de Personas Down IAP, who were diagnosed with Down Syndrome through a karyotype test. These 34 people were divided in three groups (Table 1) based on qualitative appreciation criteria with respect to their degree of social integration, as follows:
A) Group I, students with higher behavioral problems.
B) Group II, students with mild behavioral problems.
C) Group III (control), students without behavioral issues.

**Table 1. Distribution of study groups according to their age.**

| **AGE (years)** | **Number of subjects** | | |
|---|---|---|---|
| | **Group I** | **Group II** | **Group III (control)** |
| 0-3 | 1 | 3 | 1 |
| 4-7 | 1 | 1 | 3 |
| 8-12 | 3 | 2 | 2 |
| 13-17 | 2 | 2 | 4 |
| 18-25 | 2 | 1 | 1 |
| 26-36 | 0 | 2 | 0 |
| 37-60 | 2 | 1 | 0 |

A different food plan scheme was assigned to each group, as follows:
*Group* I: The intake of lactic protein (milk and its derivatives), gluten (wheat and its derivatives), high glycemic index sugar (refined sugar) and proteins from red meats (pig, cow, lamb, veal, kid and their by-products) was removed. These were replaced with casein-free milk serum (Bless® milk), low glycemic index sweetener (Super Life ®), products with alternative flours to gluten and white meat or plant proteins. The diet was supplemented with the intake of 23 grams of the food supplement of the present invention, twice a day (one in the morning and one in the afternoon), dissolved in 250 mL of casein-free milk (Bless® milk).
*Group* II: The intake of red meat and milk and its derivatives was controlled during the stay of said subjects at the Foundation CTDUCA, and they were replaced by plant-origin similar products such as soy meat and soy milk. The remainder diet was free as for products and schedule. The diet was supplemented with the intake of 23 grams of the food supplement of the present invention, twice a day (once in the morning and once in the afternoon), dissolved in 250 mL of soy milk.
*Group III* (control group): Without nutritional restrictions or supply of any intake of the food supplement.

People with Trisomy 21 (Down Syndrome) were evaluated with measurement tools or techniques in the sociological, cognitive, physiological and polysomnographic aspects. The analysis of the population was carried out with cross and longitudinal cuts, since besides evaluating the above-indicated aspects in each subject of the study population, comparisons were also made among subjects of similar ages in the diet groups in which the population was divided.

In the physiological aspect, the object of the study was to show if the food supplement of the present invention had the ability to contribute with the necessary nutrients with specific bioavailability to the particular metabolic needs, so that people with Trisomy 21 were maintained in ideal biochemical levels with reference to a scale of values determined for people with this condition, as well as to monitor neurotransmitters production and use biochemical processes. Moreover, the incidence of the selected elements in the diet in every population sector in the improvement of their hypotonic and peristaltic condition was investigated, and their nutritional health state and adaptation of weight and size were evaluated.

To this end, blood chemistry of 27 elements, serotonin in serum, total plasma catecholamines and coprology tests were made and a registration of anthropometric measures was followed.

With respect to the sociological aspect, a cross cut, holistically focused, etnographic-type qualitative-exploratory study was made, in which evidences were obtained to analyze the level of social insertion people with Down Syndrome have, and to document the modification of said conditions in the framework of the nutritional intervention and the intake of the product of the present invention. The information was collected at four times: first, by means of a multiple-option questionnaire as an introduction form to the study; second, in two semi-structured interviews with close relatives; third, with key subjects, professors belonging to the institution, and people of the close social environment with open questions; and fourth, with a final questionnaire. The interviews were transcribed and the data analyzed by means of the item technique and the content analysis technique proposed by Cáceres Mountain et al. (Diseños experimentales de series temporales. UNED. Spain, 2007).

In the cognitive aspect, the indicators to measure advances in the upper and behavioral cognitive abilities of the subjects enrolled in the study were oriented to four basic aspects of behavior and learning processes: kinesiology, attention, communication and nutritional regularization.

A longitudinal, qualitative, testimonial research was carried out, based on the analysis from a psychological point of view (cognitive) of the collected interviews in the sociological area framework of the project. In such way, the obtaining of objective testimonies related to the advances in the subjects with Trisomy 21 (Down Syndrome) was assured, from their relatives and close friends.

Finally, in order to evaluate modifications in the architecture of the sleep in the studied people, Complete Polysomnography Recordings (PSG) were carried out in the Clínica de Trastornos del Sueño de la Facultad de Medicina de la Universidad Autónoma de Mexico (Faculty of Medicine Sleep disorders clinic of the Mexican Autonomous University). These tests consisted in recording the different physiological parameters: encephalographic activity (monopolar derivations C3-A2, C4-A1, O1-A2 and O2-A1), ocular movements, muscle tone, cardiac frequency, nasal and oral air flow, thoracic and abdominal respiratory effort, snoring, blood oxygen saturation, limb movements and body position.

In this specific area of study, the population under study had a different segregation from the remainder of the areas due to the behavior of the sleep stages with respect to age. Based on the recommended standards to analyze and to compare the results of the PSG studies and to the changes due to the ontogenics of the sleep, the subjects were divided in 2 groups, the first (group A) was formed by people older than 13, while the second (group B) was formed by people under 13, according to the data shown in Table 2:

**Table 2. Groups of people with Down Syndrome for PSG testing**

| **Group A** | | | | |
|---|---|---|---|---|
| | n | Average age (years) | SD | Age range (years) |
| Men | 8 | 22.08 | 12.98 | 13-51 |
| Women | 6 | 30.2 | 8.59 | 20-42 |

| **Group B** | | | | |
|---|---|---|---|---|
| | N | Average age (years) | SD | Age range (years) |
| Men | 11 | 6.96 | 3.25 | 2-11 |
| Women | 5 | 6.95 | 4.15 | 3.3-11.6 |

The data obtained from said indicators were further compared to databases property of the Faculty of Medicine Sleep disorders clinic of the Mexican Autonomous University, from people with gender and age affinity but in neurotypical or healthy subjects (control group). In such manner statistical-type relationships could be set, which allowed knowing the sleep quality of people with Down Syndrome conforming the study population.

The results of the tests performed are shown below.

### Physiological aspect

Blood chemistry tests and serotonin and total catecholamines level determination in blood permitted to observe that the numerical results of the three study groups remained within the range from optimum to normal of the blood chemistry indicators, thereby showing the ability of the food supplement of the present invention to effectively and efficiently supply the nutrients removed from the diet of the people enrolled in the nutritional intervention plans, without deterioration of their body optimum levels and without representing an additional load damaging the correct operation of vital organs such as the liver and the pancreas, exempt of preexisting pathologies. In other words, the food supplement indeed has the ability to maintain a state of adequate nutritional balance favoring the correct metabolic and biochemical operation of the people with Trisomy 21 who consumed it.

Likewise, it was also possible to observe that groups I and II having consumed the food supplement of the present invention showed relevant additional results to the study.

In the case of the proteins, despite limitation in the red meat intake, not only does the food supplement supply an adequate quantity thereof, but also the necessary quality so that the body of the people with Trisomy 21 may absorb and use them.

On the other hand, it is common that people with Trisomy 21 show creatinine under-values (particle that contributes to muscular development) to that level considered as "normal" for neurotypical people, which associates to the muscular hypotonia inherent to Trisomy 21. In this sense, although people in group III did not have any nutritional restriction, 60% of the cases showed under-values to the normal level, while people in groups I and II which consumed the food supplement are in an optimum level in 50% of the cases, still considering that group I cannot eat red meat.

With respect to cholesterol, people in groups I and II showed more acceptable values with respect to those of group III, thereby allowing improving the heart condition of people with Trisomy 21 (Down Syndrome) who are highly prone to develop heart diseases. These results are coherent with the results obtained from the study regarding the triglyceride levels in the blood of the population under study, in which case the obtained values are within the normal range.

On the other hand, the results of the total blood serotonin and catecholamines determinations are not conclusive under the analysis models described in the methodology. However, according to testimonial evidences, the increase in the serotonin level, added to the balance in the dopamine level, reached by people who consumed the food supplement explains the receptive behavior and the advance in the cognition processes discussed below.

Also, the coprology analysis indicators offering the most relevant information regarding the hypotonic and peristaltic condition of the studied subjects, and of the general condition of the digestive tract, are the consistency, macroscopic blood and nutritional traces.

At the end of the study, 70% of the subjects in group I showed paste-like stools due to the intake of prebiotic fiber in the food supplement of the present invention. This reflects a better intestinal motility and a decrease of colitis, thereby helping to favor nutrient and mineral absorption in the body. As for group II, which consumed prebiotic fiber from the food supplement, at the term of the study it was seen that 56% of individuals showed a consistency indicative of a constant intestinal movement. With respect to group III, 73% of the boys showed paste-like stools, indicating a constant intestinal movement, which may be associated to poor fatty acid metabolism, rather than an efficient digestive efficient or nutritionally permissive process.

Now, regarding the evident or macroscopic presence of blood in stool, a lack of this element at the end of the study was seen in the results of the subjects in group I. In group II, on the other hand, at the end of the study the problem of macroscopic blood presence in stool was corrected, which was observed three months before, while the element continues being present in the subject in group III during the whole period of study. The above supports that the fibers given by the food supplement of the present invention have the proper bioavailability to strengthen the symbiotic system between the intestinal flora and fauna.

It is known also that people with Down Syndrome tend to reject the intake of prepared-food and to accept that of industrially-processed food, due to the small challenge on chewing the later offers (Abanto, J. et al. 2011. "Medical problems and oral care of patients with Down Syndrome: A literature review". Special Care in Dentristy. Volume 31. 6:197-203), thereby explaining that people in diet groups I and II, whose nutritional intervention is more severe, showed more food traces in the stools (50% and 33%, respectively), than those thrown by the subjects in group III (27%) having no restrictions. However, it is worth considering that although industrialized-food leave no traces in the stool, its nutritional contributions is low.

Regarding anthropometric measurements, and more specifically, growth (understood as the height reached by the subject during the test period, i.e., 1 year), 82% of the subjects conforming group I increased an average of 1.30 cm in size, 58% of the subjects in the diet group II grew an average of 1 cm, while in group III only 18% of the subjects showed an advance in this sense, with an average of 0.60 cm. These results show that the present food supplement supplies the adequate mineral dose to promote correct bone development, resulting in growth of the people.

Referring to the Body Mass Index (BMI), the assessment of the nutritional condition of the population under study was carried out based on the standards established by the World Health Organization. At the end of the period of study, 55% of the population in group I maintained a BMI within the range considered as normal, in the same way that the amount of pre-obese, overweight and thin subjects remained the same. In group II a subject changed his BMI from normal to overweight, impacting the dynamism of indicators, from 67% in normality in the middle of the study, to 50% at the end of the study, and from 17% of overweight subjects in the middle of the study to 33% at the end of the study; the BMI of people with pre-obesity was maintained constant, in 17%. In group III no variation was seen in the indicators in the same period: 73% of the population qualifies in the BMI normal evaluation, 18% are and remain overweight, and 9% are pre-obese. Generally, it may be considered that the food supplement does not affect the weight of the one intaking it, and the fact that a subject in diet group II has shown a variation in the population percentages of proper weight may be attributed to the soy-enriched diet of this group.

Another assessment with respect to the weight and size ratio in each subject was carried out using the reference tables from the Mexican Official Rules NOM-008-SSA2-1993 and NOM-174-SSA1-1998. As a result, a general improvement in the weight/size ratio in the subjects in diet group I was seen, since during the period of study the malnutrition in all its magnitudes was eradicated, and the percentage of overweight people was reduced. At the end of the study, 55% of said group I was found in a satisfactory size to weight ratio, while at the start of the study, only 27% was in a satisfactory weight/size ratio, which may be attributed to the fact that the food supplement of the present invention has a direct effect on the sensation of satiety helping to fight overweight, but without affecting the nutritional health of the people intaking it. Regarding group II, the nutritional state of the subjects remained stable in the period of analysis (unchanged), while the weight/size ratio was also maintained unchanged, i.e., in 58% of the subjects with normal conditions at the start and at the end; in 25% of the overweight subjects at the start and at the end; and in 17% of the obese subjects at the start and at the end. On the other hand, the results of the group III reinforce the findings in the analysis of blood chemistry and coprology tests, in the sense that the subjects having no nutritional intervention plan and who do not take the food supplement are more prone to have nutritional disorders, since the malnutrition remained during the course of the study, although changed from mild to low, and the obesity had a substantial advance passing from 9% to 18%, suggesting an unstable nutrients supply.

### Sociological aspect

As mentioned above, the sociological research was focused in revising how the perception of future expectations, social context and family context was changed, on the families with a member with Down Syndrome who was subjected to the nutritional intervention model and to the intake of the food supplement of the present invention.

The answers to the exploratory questionnaires showed some considerable changes due to the nutritional intervention. At the behavioral level, the parents of the subjects in groups I and II perceived a tendency to be more tranquil in their children, with less violent and aggressive events, giving them more relaxation times and increasing their confidence to carry out complex, social activities, such as going to a restaurant with no fear of misbehaviors. Families of the subjects in groups I and II, who changed their nutritional habits as a result of the study and in support to their children were the ones having a better perception with respect to the changes at the physical level (weight loss, greater mobility, greater satiety) in their children, while the families showing low or no interest in the behavior and cognitive conditions in their children, did not perceive any change (3 cases). Families who perceived greater changes in their children, also modified the expectations in their future life and prefigured a better quality of social and family life. The major change noted by the parents was in the cognitive area, by perceiving that people in groups I and II showed higher concentration, attention and disposition in tasks that before were indifferent, difficult and complicated thereto, compared to subjects in group III.

### Cognitive aspect

Changes observed in the subjects in groups I and II were classified in two great areas: behavioral and higher cognitive abilities.

Behaviorally, it was possible to appreciate that 70% of the population in both groups had clear advances in the anxiety management in aspects such as satiety, control and reduction of anger and aggressive crisis. Moreover, it was seen that 60% of individuals studied increased their tolerance levels and substituted disruptive behaviors by socially acceptable attitudes, while 100% of people in both groups showed a better adaptation to social systems, thereby enjoying of a quality and more intense interaction with the family and people around.

Regarding higher cognitive abilities, the testimonial information analysis about the progress was carried out from four points of view: kinesiology, nutritional regularization, communication and attention, as was mentioned above.

Kinesiologically, at the term of the study, 90% of the subjects studied showed clear advances in the performance of their fine motor abilities, added to 50% perfecting their gross motility.

80% of the subjects in groups I and II showed progress in the control of anxiety producing a compulsive food intake, and about 40% of said subjects made voluntary feed adjustments, not only as a result of the recently learned ability to follow the satiety feeling, but understanding, based on the experience, the effects the food supplement have on their state of health.

Likewise, 100% of the population in groups I and II showed progresses in the communication aspect. In this sense, although the subjects without verbal language failed in developing it, the establishment of communication and interaction self-codes with the people around them was seen, while the subjects already having a verbal language perfected and improve the reading-writing comprehension and in meaning retention to give proper answers.

Similarly, 100% of the subjects in both groups showed progress related to the attention, showing more often the understanding of simple instructions and the following execution thereof, in some cases with help to conclude them, and in other cases with autonomy development for their completeness.

Regarding the population in group III, there were no behavioral changes nor in the higher cognitive abilities thereof.

### Polysomnographic aspect

As mentioned above, in order to study this aspect, it was necessary to form 2 groups (A and B) based on the subjects age, since the sleep ontogeny indicates that known sleep parameters values are set around the age of 13. Thus, the comparison of people with Down Syndrome with the healthy subjects group (control group) is methodologically more reliable.

As a result of the carried out tests, differences regarding weight in the subjects and the control group were found. In the group younger than 13 (group B) a higher weight in men compared to women was seen, this difference being greater with respect to the control group. The above explains the presence of snoring and sleep apnea in the male group, since it is known that overweight is a risk factor for the presence of breathing disorders induced by sleep.

With respect to height, people showed smaller size than the control group, in an age-independent manner, which increases the BMI and also influences in the presence of breathing disorders induced by sleep.

The lapse of the PSG studies was the same for the 3 study groups, then the differences observed in the different physiological parameters studied are a result of the expression of the sleep physiology of each group.

Compared to their gender and age pairs, people with Down Syndrome in groups A and B slept less time and spent more time in light sleep formed by stages 1 and 2, then causing a reduction in stage 3, referred to as having restoring functions from the physical point of view. Also a Rapid Eye Movement (REM) sleep-stage decrease was seen, which known function is to restore the higher mental functions.

Likewise, the presence of the obstructive sleep apnea syndrome was observed in people with Down Syndrome, being mild in people under 13 (group B), while being severe in the subjects older than 13 (group A). It is worth to note that said obstructive sleep apnea syndrome produces alterations in the sleep structure, producing, in turn, cognitive deterioration.

Comparing collected data in the baseline Complete Polysomnographic Recordings of the population of study with Down Syndrome, versus the control studies after nutritional intervention, it could be noted that -being each subject his own control in contrast to their baseline data- the obstructive and central apnea frequency considerably decrease, also, shorter events of movements related to anxiety during the sleep were reported (such as Incorporations, somnambulism, involuntary movements associated to breathing obstructions), the reflux events substantially decrease, and the permanency in the stages of sleep III and IV substantially increases, associated to learning consolidation and to the restoring functions of the body functions.

From the above results, it may be appreciated that the food supplement of the present invention has a constant and proper biochemical supply favoring a nutritional balance, promotes peristalsis, helps ameliorating hypotonia inherent to people with Trisomy 21 or Down Syndrome, and coadjuvates to the correct development of these people.

### Example 2. Effect of the food supplement in the formation of dendritic spines, synapse and cognitive development.

A second study was carried out in order to assess the possible effect of the food supplement of the present invention on behaviors different to those of Example 1, by exploring whether oral administration of the food supplement in mice would improve the ability of these rodents to resolve tasks related to cognitive and motor functions, and whether this is correlated with an increased number of dendritic spines and synapses.

To this end, two groups were formed, each one with 3 3 weeks old CB57BL6/J male mice, which remained in individual polycarbonate cages at a temperature of 20±2° C and a cycle of light/darkness of 12 hours with lights on from 7:00 to 19:00 hours. All the tests were carried out during the light-time, and the mice had free access to food.

In group 1 the mice received a dose of 80 mg/mL (equivalent to the dose administered to the subjects in Example 1) of the food supplement of the present invention, twice a day, for 30 minutes over 3 months. To assure the intake of the food supplement, the mice were water-deprived.

In group 2 (control group), there was no administration of the food supplement.

For the behavioral tests, behaviors related to learning and memory through the Novel Object Recognition (NOR) and the Y-shape Aquatic Labyrinth (YAL) tests were analyzed.

For the NOR test, the mice of group 1 fed with the food supplement during 4 and 6 weeks, and group 2 (control), were individually exposed in an open field polycarbonate box (55 cm X 55 cm X 25 cm height) for 10 minutes, during 3 days, to allow a free exploration (stage of habituation). On day 4, two identical objects were placed in the open field box and the mouse was allowed to explore for 10 minutes (stage of familiarization). Subsequently, the mouse was withdrawn from the box for 10 minutes and then reintroduced in the open field box, but one of the familiar objects was replaced by a new one (test stage) for 10 additional minutes. Normally, the rodents tend to spend more time exploring a new object than a familiar one. The time and the discrimination index dedicated to explore each object were quantified.

For the YAL test, the mice of group 1 having consumed the food supplement for 6 weeks, and the mice of group 2 were exposed to a white acrylic YAL (each arm measures 30 cm length X 5 cm wide and 16 cm depth) full with water. One of the arms included a platform allowing them to remain out of the water. After the period of habituation, the mouse was removed from the labyrinth and returned to its box, and 24 hours later the mouse was exposed again to the YAL, the time spent to find the platform being quantified.

Likewise, the open field test (OFT) was used to assess the anxiety levels and the locomotive activity in the groups 1 and 2 mice. The OF in a transparent acrylic box (55 cm × 55 cm × 25 cm), divided in 25 equal quadrants. To carry out the test, the animals were located at the center of the box, and the number of quadrants horizontally crossed by the animal for 5 minutes (crossing), the total distance traveled and the total number of movements were quantified. Also, defecation and sprucing up were assessed, which are anxiety associated behaviors.

Likewise, the Elevated Plus Maze (EPM) is a paradigm to measure anxiety levels. The EPM is made of acrylic with two open arms (30 cm length X 6 cm wide), and two closed arms (30 cm length X 6 cm wide and a 15 cm height dark wall) lifted at 40 cm from the floor. Rodents generally prefer closed arms compared to open arms, visiting more often and significantly longer time in the closed arms. The number of visits and the amount of time spent in the open arms is considered as an anxiety index. The anxiety levels should correspond to the amount of time spent in the closed arms compared to the open arms. For this test, the mice were placed at the central area to allow the access to the 4 arms. The chosen behavior was observed by 5 minutes and the number of visits to each arm, the time spent in each arm as well as the central area were quantified. It is worth mentioning that the behavioral assays were video-recorded.

On the other hand, the analysis of the dendritic spines was carried out through the fast Golgi assay (Colín Barrenque et al., 1999), for which the groups 1 and 2 mice brains were previously removed and fixed with 4% paraformaldehyde in 0.1 M phosphates buffer. Subsequently, the brains were submerged in a 2.7% potassium dichromate and 0.3% osmium tetraoxide solution for 7 days. After this period the brains were placed in a 0.75% silver nitrate solution for one day. The brains were sliced at 90 µm, which were developed and mounted in covered slides.

For the quantitative analysis, only neurons showing a complete impregnation of the dendritic tree and relatively isolated from adjacent neurons were selected. The number of spines of a neuron was quantified manually and normalized in segments of dendrites of 20 µm length, and the results were expressed as the number of spines by 20 µm.

After administering the food supplement of the present invention to the mice of group 1, twice a day, during 3 months, statistically significant changes in the mice of group 1 weight and size (which were assessed weekly) with respect to the control mice (group 2) were not found. Likewise, as the food supplement administered to group 1 includes sugars and flavorings in its composition, and in order to determine that these compounds did not produce a preference to the flavor manifested as an increased intake of said food supplement, the liquid intake was measured daily, over 40 days. The results indicated that the mice consume the same volume of liquid during this period. Similarly, it was assessed that the presence of diverse minerals, vitamins and serum concentrated protein in the food supplement did not stimulate the satiety and influence in the amount of food consumed by the mice, then quantifying the daily pellet intake over 40 days. The results indicated that the food intake was not modified for the intake of the food supplement of the present invention.

With respect to the behavioral tests, particularly related to the Novel Object Recognition task (NOR), the mice in group 2 spent more time observing the novel object compared to the mice in the control group, while the results for the discrimination index (DI) suggest that the present food supplement improves the recognition memory, achieving an ID of about 54% in the Group 2 with respect to an ID of 41% in the control group at week 4, as well as an ID of 45% in group 2 with respect to an ID of 18% in the control group, at week 6.

On the other hand, the obtained results in the Y Aquatic Labyrinth (YAL) suggest that the intake of the food supplement does not have a significant effect on spatial memory processes.

Regarding the Open field test (OF), the mice of group 1 subjected to a CA show a significant decrease in the sprucing up and defecation (behaviors related to an anxiety state) compared to group 2 (control). Said decrease was of 46% less sprucing up and 33% less defecation for the mice of group 1. Besides, the mice of group 1 showed a tendency to reduce their anxiety levels, which was seen as an increase in the number of crossings in the OF, the animals staying more time at the center of the box (inner time) and reducing the time spent near the box walls (outer time). It is worth pointing out that these behaviors are associated to an increase in the motor activity.

In the Elevated plus maze (EPM) test, it was found that the mice of group 2 stay 72% more time in the open arms compared to the control group, and they increase in 40% the frequency they show their heads through the open arms edges with respect to the control group, thereby indicating a decrease in the anxiety levels of the animals. Both data speak of a greater anxiety control and of a renewed sense of self-confidence and exploration. Finally, the results of the dendritic spines analysis suggest that the intake of the food supplement produces an increase in the diameter of the dendrites, as well as in the number of dendritic spines, which would favor the development and maintenance of neuronal circuits. Likewise, it was observed that the mice of group 1 (which consumed the food supplement and were also subjected to a behavioral stimulation process), showed an additional increase in the number of dendritic spines.

According to the above, it can be seen that the food supplement of the present invention improves certain behaviors associated to object recognition memory in mice, but it has no significant effect on spatial memory; this suggests that the food supplement may have a differential effect on the diverse types of memory. On the other hand, the intake of the food supplement reduces anxiety associated behaviors, then, this product may be used as an anxiolytic which due to its nature would not produce any dependence. The behavioral improvements observed in mice would be associated to an increase in the diameter of the neuron dendrites and an increase in the number of dendritic spines, thereby producing stronger and more stable neuronal circuits.

It is known that all cerebral activity in the human being is directly related to the amount, intensity and type of synapse occurring among its neurons through the dendritic spines, such that people with some degree of mental disability have substantially thinner dendrites (smaller diameter) that "neurotypical" people, as well as less spines, or immature spines called "filopodia".

While the trisomic condition either in pair 21 (Down Syndrome) or in pair 18 (Autism) are chromosomal manifestations and will accompany the subject during his/her whole life, it is also right to affirm that support to favor the development, maturing and communication among the neurons, would represent a substantial enhancement in their quality of life thereby benefiting the development of, among others, cognitive, behavioral, social and emotional abilities.

As for the people with ADD or ADHD, this condition is explained by neuronal communication abnormalities found in the different areas of the brain, either by an excessive electricity flow or because of immaturity in any area. A development and performance benefited from the cerebral microarchitecture by an increase in the dendritic branching directly contributes in the regularization of said situations.

### Example 3. Food supplement assessment in people with Trisomy 21 (T21), Autism Spectrum Disorders (ASD) and Attention Deficit Disorder with or without hyperactivity (ADD or ADHD)

In order to assess the performance of the food supplement of the present invention in people with ASD and ADD or ADHD, as well as to demonstrate the results in people with T21 shown in Example 1, a longitudinal prospective cut study was carried out in said populations, with holistic approach, which lasted 12 months.

Study populations consisted of 24 subjects with T21, 26 subjects with any of the ASD and 20 subjects with ADD or ADHD (hereinafter, ADD and ADHD will be referred to as ADD).

In each case, the populations were divided into three groups, according to the following:
Pilot group (D + C): its members were assigned a recommended nutritional plan, administered 46 grams daily, divided in two 23 grams doses, one for breakfast and the other before the night sleep.
Diet Group (D): its members adopted only the recommended nutritional plan and did not have any nutritional enrichment.
Control or Witness group (T): its members did not adopt the recommended nutritional plan, neither took the food supplement of the present invention.

The nutritional plan consisted in the removal of products containing gluten, casein, soy, fermentable sugars (such as sucrose, table sugar, etc.) as well as the reduction in red meat intake.

In the performed experiment, diverse parameters related to biomedical, etnographic, neuropsychological and sleep physiology were measured. The anthropometric measurements were made every 3 months and the shown results correspond to the average values at the end of the study.

In the biomedical aspect, the intention was to establish the metabolic clinical condition of the gastrointestinal tract (disbiosis), as well as the nutritional condition, of feed allergies and sensitivities, of the population forming each group.

With respect to the ethnographic aspect, the object of the study was to revise the caused impact from the diet and the intake of the food supplement in the quality of life of the people with T21, ASD and ADD, as well as that of their families.

Regarding the neuropsychological aspect, the cognitive and emotions recognition abilities in people with the above-mentioned sufferings were explored, before and after intaking the food supplement and from following the nutritional plan.

In the sleep physiology aspect, the intention was to corroborate the benefit in the balance in the sleep architecture related to the intake of the food supplement of the present invention by people with T21, ASD and ADD, and to document through neuropsychological techniques the physiological changes produced by said supplement, as well as by the diet, in these subjects.

The obtained results in the tests for each of the above-mentioned areas are shown below.

### Example 3A. Biomedical aspect

To each one of the groups, blood biometric, blood chemistry, reactive C protein, homocysteine, plasmic cortisol, thyroid profile, coprology and 3-sample coproparasitoscopic, organic acids, gluten and casein peptides, IgG and Candida albicans food allergies and sensitivities studies were made. The results at the baseline (B) and post-intervention (PI) are shown below, where the percentage (%) of change is a statistical value to assess the number of times a reference value was increased or decreased. To this end, a value of 1 or 100% was assigned to the reference limit-values, and the percentage in which the average results approach said value was assessed. It is worth mentioning that the nature of said indicator is completely related to the nature of the parameter being evaluated.

In the first place, as a result of these tests it could be demonstrated that at the baseline, i.e., at the start of the study and before the subjects received the food supplement and/or the diet, the three populations shared diverse characteristics such as allergies and sensitivities to various food, depressed immune system, disbiosis, etc.

In Table 3 the values obtained for the disbiosis markers are shown in all three populations, at the start (B) and at the end of the study (PI).

**Table 3. Disbaiosis markers in people with T21, ASD and ADD.**

| **CASEOMORPHINE** | **T21** | | | **ADD** | | | **ASD** | | |
|---|---|---|---|---|---|---|---|---|---|
| | **T** | **R** | **D + C** | **T** | **R** | **D + C** | **T** | **R** | **D + C** |
| **R** | 0.94 | 1.97 | 2.5 | 3.68 | 5.74 | 2.92 | 8.23 | 3.9 | 4.41 |
| **PI** | 1.34 | 0.91 | 1.69 | 4.35 | 4.58 | 4.97 | 1.82 | 2.53 | 1.57 |
| **% of change** | -42.55 | 53.81 | 32.40 | -18.21 | 20.21 | -70.21 | 77.89 | 35.13 | 64.40 |

| **GLUTEOMORPHINE** | **T21** | | | **ADD** | | | **ASD** | | |
|---|---|---|---|---|---|---|---|---|---|
| | **T** | **R** | **D + C** | **T** | **R** | **D + C** | **T** | **R** | **D + C** |
| **R** | 0.11 | 0.95 | 0.17 | 1.34 | 0.51 | 0.51 | 0.16 | 0.38 | 0.36 |
| **PI** | 0.19 | 0.25 | 0.28 | 0.24 | 0.56 | 0.98 | 0.24 | 0.66 | 0.33 |
| **% of change** | -72.73 | 73.68 | -64.71 | 82.09 | -9.80 | -92.16 | -50.00 | -73.68 | 8.33 |

Caseomorphine and gluteomorphine may be indicators of an altered operation of the digestive system. This type of alterations have in common an enzyme dysfunction, particularly a deficiency in bipeptidyl/dipeptidase IV (DDPIV) to properly digest casein and gluten.

Regarding caseomorphine for the population with ADD, all three groups showed high levels in the baseline results, observing that only the subjects in group D had a 20% decrease in the levels of this peptide. With respect to gluteomorphine, its levels were out of the reference interval only in group D, being this the only group with a gluteomorphine level reduction, while groups D and D + C increased their levels above the reference interval, which may be associated to a lack of diet attachment by the subjects, i.e., they ate food containing gluten.

The population with ASD showed a different behavior, the baseline results for caseomorphine for all three groups were far above the reference interval, and in the PI results a decrease up to 70% in the three groups was seen, although without reaching the reference interval. The fact that the caseomorphine levels in group T also decreased may be explained since there were patients with milk restrictions, although they had to follow a regular diet. The baseline results for gluteomorphine are low, within the reference interval, but in the PI results both groups T and D increased their gluteomorphine levels, while only group D + C decreased its levels.

Finally, the population with T21 showed caseomorphine levels above the reference interval in all three groups; in the PI results group T increased its levels and groups D and D + C decreased their levels, although they did not reach the reference interval levels. With respect to gluteomorphine, the results were more similar than they were for the population with ADD, although in this case the increase in gluteomorphine levels was not above the upper reference interval.

These results show that the three groups at the start of the research have disbiosis problems, and it was shown that the intake of the food supplement according to the present invention improves the intestinal conditions of the subjects.

Now, in Table 5 the measurement results for 5-hydroxyindole acetic acid ((5-HIAA) in the three populations are shown.

**Table 4. Levels of 5-hydroxyindole acetic acid ((5-HIAA) in people with T21, ASD and ADD.**

| **(5-HIAA)(Serotonin) nmol/mol Creatinine** | **T21** | | | **ADD** | | | **ASD** | | |
|---|---|---|---|---|---|---|---|---|---|
| | **T** | **R** | **D+C** | **T** | **R** | **D+C** | **T** | **R** | **D+C** |
| **R** | 0.45 | 0.36 | 0.35 | 0.71 | 0.63 | 0.76 | 0.43 | 0.68 | 0.6 |
| **PI** | 2.03 | 1.44 | 0.96 | 1.51 | 0.69 | 1.2 | 1.25 | 4.82 | 2.84 |
| **% of change** | -351.11 | -300.00 | -174.29 | -112.68 | -9.52 | -57.89 | -190.70 | -608.82 | -373.33 |

As can be appreciated in Table 5, 5-HIAA levels are decreased in all the groups, possibly indicating a serotonin neurotransmitter synthesis deficiency, since it is a neurotransmitter metabolite in which the decreased values are associated to depression. The values corresponding to PI show that in the population with T21 and ASD a considerable metabolite increase was generated, mainly in group D and in a lesser extent in group D + C. The scarce presence of 5-HIAA at the baseline is attributed to the low serotonin production due to the very high intestinal disbiosis, since 95% of the body serotonin used in the body is produced at the bowel. Regarding the substantial increase in this metabolite levels in groups D and D + C in the PI, it is important to point out that a high concentration thereof is related to a good serotonin production, and also with a low use of this neurotransmitter. This explains why people in group D + C had lower 5-HIAA levels in the PI, compared to people in group D, although both groups produced a suitable serotonin level, people in group D + C not only produced it, but they also used it for the basic body functions, where the serotonin plays a predominant role.

In the population with ADD, levels in the PI increased but not in the same way seen in the other two groups.

On the other hand, the nutritional indicators shown in Table 5 were evaluated in the three populations.

**Table 5. Nutritional indicators in people with T21, ASD and ADD.**

| | **TOTAL CHOLESTEROL mg/dL** | | | | | | | | |
|---|---|---|---|---|---|---|---|---|---|
| | Recommended: <200 | | | | | | | | |
| | **B** | | | **PI** | | | | | |
| | Total in the population | | | R | | | D+C | | |
| | **T21** | **ADD** | **ASD** | **T21** | **ADD** | **ASD** | **T21** | **ADD** | **ASD** |
| *Average Result* | 171 | 153 | 181 | 167 | 172 | 177 | 164 | 145 | 184 |
| *% population^{a}* | - | - | - | 57% | 50% | 64% | 60% | 80% | 70% |

| | **ASCORBIC ACID (Vitamin C) (nmol/mol Creatinine)** | | | | | | | | |
|---|---|---|---|---|---|---|---|---|---|
| | *Reference Ranges: 10-200 nmo*/*mol Creatinine* | | | | | | | | |
| | **B** | | | **PI** | | | | | |
| | Total in the population | | | R | | | D+C | | |
| | **T21** | **ADD** | **ASD** | **T21** | **ADD** | **ASD** | **T21** | **ADD** | **ASD** |
| *Average Result* | 67.1 | 27.66 | 35.89 | 31.9 | 29.5 | 12.52 | 90.4 | 75.78 | 229 |
| *% population^{b}* | - | - | - | 57% | 40% | 45% | 60% | 55% | 30% |

| | **VITAMIN B6 (Pyrodoxine hydrochloride)** | | | | | | | | |
|---|---|---|---|---|---|---|---|---|---|
| | Recommended: <53nmol/mol Creatinine | | | | | | | | |
| | **B** | | | **PI** | | | | | |
| | Total in the population | | | R | | | D+C | | |
| | **T21** | **ADD** | **ASD** | **T21** | **ADD** | **ASD** | **T21** | **ADD** | **ASD** |
| *Average Result* | 5.45 | 6.14 | 10.8 | 9.7 | 5.63 | 22.3 | 12.5 | 12.42 | 19.3 |
| *% population^{b}* | - | - | - | 86% | 60% | 73% | 70% | 91% | 80% |

| | **COENZYME Q10 (nmol/mol Creatinine)** | | | | | | | | |
|---|---|---|---|---|---|---|---|---|---|
| | *Reference Ranges:* 88 *nmol*/*mol Creatinine* | | | | | | | | |
| | **B** | | | **PI** | | | | | |
| | Total in the population | | | R | | | D+C | | |
| | **T21** | **ADD** | **ASD** | **T21** | **ADD** | **ASD** | **T21** | **ADD** | **ASD** |
| *Average Result* | 26.53 | 24.8 | 37 | 23.75 | 18 | 34.5 | 28 | 40.43 | 85 |
| *% population^{b}* | - | - | - | 29% | 40% | 18% | 20% | 64% | 30% |

| | | | | | | | | | |
|---|---|---|---|---|---|---|---|---|---|
| a=% population improving the cholesterol balance b =% population with a concentration increase | | | | | | | | | |

As can be seen, the three populations substantially improved their cholesterol levels after the treatment by getting closer to the recommended optimum level. More than half of the subjects in group D of the three populations had improvements, being the most benefited the ASD group, clearly showing that the intake of the food supplement favors a better balance in total cholesterol levels, with improvements in the majority of the three populations in group D + C. This result is interpreted as if the subjects exceeding a total cholesterol recommended level reduced its concentration, while the subjects below the optimum level, increased it.

With respect to vitamin C, it could be seen that the majority of the populations had extremely low levels of this vitamin at the start of the study. However, people in group D + C of the three populations at the end of the intervention period, had a substantial improvement in their vitamin C levels.

Referring to vitamin B6, it was observed that the populations with T21 and ADD share low levels of this metabolite, while the population with ASD has higher baseline levels, however the three populations' levels are considered low. In the present study, it was found that the subjects with an excessive growth of *Candida albicans* do not metabolize this vitamin, since the byproduct of this yeast, arabinose, competes for numerous enzymes sites where the vitamin B6 works as a cofactor, blocking the action of this vitamin.

With respect to the coenzyme Q10, the obtained results showed that the three populations involved had reduced baseline levels within the reference interval (lower or equal to 88 nmol/mol creatinine). In contrast, the obtained values in the PI show clear increases in the concentration of this important coenzyme, from between 18% and 40% in group D of the three populations, but more importantly in group D + C, where the increases varied between 20 and 64%, although always within the reference interval.

As mentioned above, food allergies and sensitivities of the three populations were also assessed. In this sense, in the populations with T21 and ASD, a gluten sensitivity decrease was generally found (protein only) and whole wheat (protein, starch and germ) going from high to mild. Referring to the population with ADD, there were very low reduction percentages with respect to the high levels measured in the baseline, then the sensitivity levels remained within the high intervals.

Likewise, baseline sensitivities of the IgG type to the yeast *Candida* albicans were assessed. The results showed high levels, almost twice the set limit, for the population with T21 and ASD, in both the group D and D + C (lower or equal to 5). The PI sensitivities decreased in each case, although they were still found within the high levels, this decrease is an indicative of the reduction in the disbiosis caused by candidiasis.

Similarly, in the population with ADD the starting sensitivity level to *Candida albicans* determined in group D is high (9.06), almost twice the reference value; the PI sensibility result reflected a 17% decrease, although the levels remained in high parameters. A different behavior was seen in the group D + C, wherein the baseline result was a high sensitivity level (6.8) and after the intervention the result was a 15% reduction in the IgG sensitivity, but still remaining in a high level.

As can be seen from these results, the IgG sensitivity values for *Candida albicans* in the three populations are at very high levels, fully coinciding with the presence of chronic basal candidiasis in the subjects of every population. In this aspect, the nutritional intervention (wherein the fermentable sugars are removed and the soluble fibers are increased by means of the intake of the food supplement), is essential in the treatment of disbiosis. However, since the colonization by this yeast and related bacteria was completely compromising the intestinal tract, the intervention periods failed in eliminating the sensitivity, thereby indicating that in longer times the IgG type sensitivities may be eliminated.

### Example 3B. Ethnographical aspect

Although the affectations to people with T21, ADD and ASD are different, the present study was an etnographic-type based research in the families of people belonging to these three groups and to revise the form in which they perceive behavioral, cognitive and social modifications in the intervened subjects and how this impacts the concept of quality of life. To this end, three activities were made:
a) interviews and longitudinal-type etnographic questionnaires were applied to document from the baseline of the three populations up to their conditions one year later, in parallel, a specific indicators table was applied to verify the processes appearing in the studied population.
b) the questionnaire about Quality of Life of the University of Kansas and The Beach Center was applied, revised by Miguel Angel Verduzco and Schalock, and a group dynamics with the parents of the different groups was carried out.
c) a contents analysis was made to be able to longitudinally structure the research conclusions.

With the above, the quality of life level of the families with people pertaining to the three populations was explored regarding the three basic indicators: emotional welfare, family interaction and social welfare.

Generally, parents of groups D + C of each population stated that their children had longer concentration periods of time, better disposition to pay attention, increased their language abilities, were more sociable, got ill less, lost weight, had less episodes of disruptive behaviors, showed a greater creative and physical activity and improved their sleep quality. Similarly, parents of the people enlisted in group D of the three populations reported that their children got ill less, lost weight and have less allergies than before.

In the health aspect, in group D + C, not only weight loss of the participant was reported, but of the relatives accompanying him in the process; likewise a height increase was reported for some participants.

For the group D + C, an improvement in the family interaction was reported, as well as the interaction of the subjects at school. Likewise, in quality of life perception, families reported a greater vitality and satisfaction upon carrying out their activities.

For the T groups of the three populations, no positive modifications were seen regarding future life expectations of the families for the family nuclei, and particularly for the disabled member. No improvements in general health condition and subject's behavior were seen, neither increases in the cognitive activity indicators could be documented.

### Example 3C. Neuropsychological aspect

In order to evaluate this aspect in the population with T21, neuropsychological tests, such as Neuropsi Attention and Memory test (Ostrosky, 2003), Computerized Neuropsychological testing (Ostrosky and Spirited, 2009), Executive Functions and Frontal Lobe tests (Flowers, 2011), and Facial Expression Recognition Task with stimuli from the Be-Face Battery (Moralez, 2010) were applied.

Participants with T21 in group D + C showed changes in personal orientation, i.e., they could refer more correct personal data, as well as in visospatial memory and in information codification, having the ability to identify more elements of the figure to copy; as well as in motor abilities, being capable of making and inhibiting movements. Only in group D a benefit to recover auditive-verbal information with less intrusions of the information was found, while in group T no significant improvements were found.

In the population with ADD the assessments were carried out according to the following: a) a first assessment where the Neuropsi Attention and Memory tests, Frostig Developmental Test of Visual Perception (DTVP-2) (Hammill, Pearson and Voress, 1993), Conners Scale for Parents- Revised (Long Version) (Sattler, 2004) and EDAH Scale for the Diagnose of the Attention Deficit Disorder with or without Hyperactivity (Farré and Narbonne, 2003) were applied; b) in the second assessment the tests of Wechsler Intelligence Scale for Children (WISC-RM) (Wechsler, 1983), Neuropsi Attention and Memory, Frostig Developmental Test of Visual Perception (DTVP-2), Manifest Anxiety Scale in children-Revised (CMAS-R) (Reynolds and Richmond, 1997) were applied; and in the third assessment the carried out tests were Neuropsi Attention and Memory, Frostig Developmental Test of Visual Perception (DTVP-2), Conners scale for Parents- Revised (Long Version) and EDAH Scale for the Diagnose of the Attention Deficit Disorder with or without Hyperactivity.

For this population, according to the baseline results, no statistically significant differences were found in members performance in the instruments in the first test events, then it was concluded that the subjects were in a similar baseline and therefore the possible detectable changes in the subsequent assessments could be attributed to the treatment applied to groups D and D + C.

At the end of the three assessments, the sub-scale scoring of the Conners hyperactivity scale and the risk level remained constant between the pre- and PI in both group T and group D, but not for group D + C, where both indicators significantly decreased. Further, when comparing these among the three groups, in the PI only the hyperactivity risk level was significant. It is important to remark that group D + C showed the lowest risk level in the post-assessment, since all the participants in this group qualified without suspects of hyperactivity risk. Further, with respect to the difference between the pre- and PI, this group also resulted in a lower scoring and a lower risk level, which may be attributed to the nutritional intervention and to the intake of the food supplement according to the present invention.

Finally, in the EDAH scale, significant differences were found only between the pre- and post-assessment in the scoring of the behavioral Disorder category in group D + C, since the PI scoring was lower. Additionally, in this last evaluation, there were significant differences between the groups in the hyperactivity, behavior disorder, hyperactivity + deficit of attention categories, except for the attention deficit category. Again, the group obtaining the lowest scoring in these categories was group D + C.

In the case of the population with ASD, neuropsychological assessments were carried out by applying the IDEA inventory designed by Angel Riviere.

The results show an improvement in group D + C, where positive changes existed in communicative abilities, as well as in comprehension and anticipation in the routine of tasks; group D also showed improvements but in a lower proportion. Specifically, the participants increased their vocalizations and use of words during their communication. For those having no oral language, more consistency in the use of the communication devices was reported, having quick progress in the stages of their program, and improvements in the receptive language (comprehension of instructions).

Likewise, participants showed improvements in comprehension and sense of activities to carry out, with a better anticipation and adjusting to routine changes, in some cases without the need of specific indication, showing a little more flexibility.

### Example 3D. Sleep physiology aspect

As is well known, there are four general stages of sleep: the first three stages together constitute the light sleep or NREM (Not REM, where REM means Rapid Eye Movements), while the fourth stage is known as REM sleep. Because the REM stage is associated to the body restoring functions and to the consolidation of the knowledge acquired during the day, and people of the study populations show severe sleep disorders due to their characteristics related to neurocognitive, including craneo-facial, disorders, the subject does not reach the REM sleep stage, thereby remarking physiological depletion and cognitive and social impediments, changes in sleeping habits of the intervened subjects were studied.

Ages of the subjects in each population ranged between 3 and 15 years old, and they were paired by age and gender. The obtained results for these populations were compared to a control group consisting of a population of subjects with similar gender, age, weight and height characteristics to the population of study, but with no neurocognitive disorder and whose nutritional habits were not modified at all.

The physiological parameters measurement used to demonstrate the changes in the body physiology of the subjects with T21, ASD and ADD after subjecting to the nutritional plan combined with the intake of the food supplement, was carried out by polysomnography, at the baseline and after the intervention (PI) with the food supplement of the present invention. Briefly, the polysomnographic study consisted of continuous recording electroencephalographic activity over at least 8 hours (monopolar derivations C3-A2, C4-A1, O1-A2 and O2-A1), ocular movements, muscle tone, cardiac frequency, nasal and oral air flow, thoracic and abdominal respiratory effort, snoring, blood oxygen saturation, limb movements and body position.

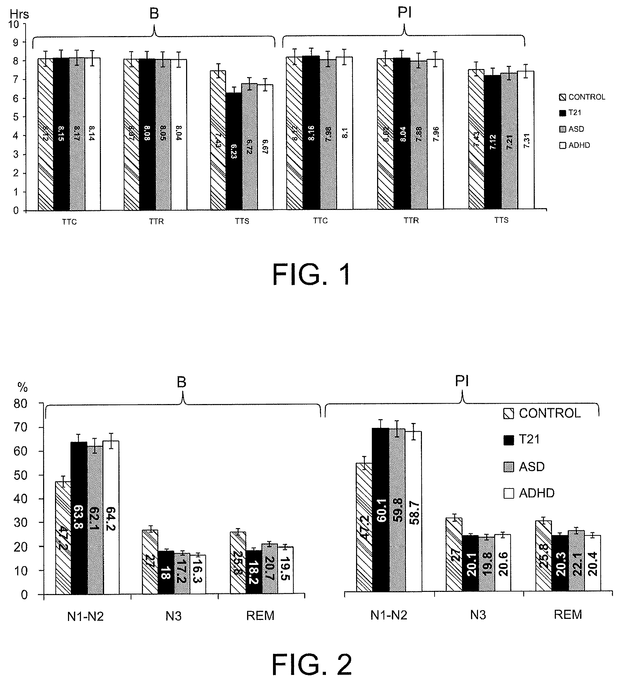

Fig. 1 shows the relationship between average values (expressed in hours) of the total time the subject spent in bed (TTB), the total time the recording lasted (TTR), and the total time of sleep (TTS). There it can be seen that the TTS substantially improved for the three populations under study, then being possible to conclude that the nutritional intervention combined with the nutritional enrichment by means of the food supplement of the present invention increases the sleep efficiency in people with T21, ASD or ADD.

As to Fig. 2, it shows the percentage relationship between the stages during the TTS, i.e., the distribution of the four sleep stages. As can be noted, subsequently to the nutritional intervention and the nutritional enrichment with the food supplement a decrease in the sleep stages 1 and 2 occurred (N1 and N2, respectively), associated to the NREM, and the permanency of the subject in stages 3 (N3) and NREM associated to the deep sleep substantially increased, thereby proving that the food supplement, combined with the nutritional plan, may have a positive impact on the cognitive and social abilities, and helps preserving the physiological and metabolic integrity in the body of subjects in the populations in question.

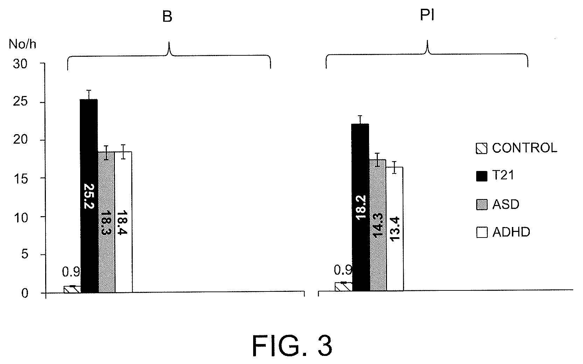

Finally, in Fig. 3 the number of sleep apneas per hour in the study populations is presented. In this figure it is possible to see a substantial decrease in the occurrence of said disorder in the subjects with T21, ADD and ASD after being subjected to the treatment with the food supplement and the nutritional plan. This decrease is important since the apnea is a disorder where breathing is interrupted, then impeding the correct oxygenation in the body to preserve its functions, mainly cerebral, and this affects duration of sleep stages.

According to the above-described, it can be seen that the food supplement for people with Trisomy 21, Autistic Spectrum Disorders and/or Attention Deficit Disorder with or without Hyperactivity, has been conceived to support nutritional therapy of these people, and it will be evident for those skilled in the art that the embodiments of the food supplement for people with Trisomy 21, Autism Spectrum Disorders and/or Attention Deficit Disorder with or without Hyperactivity, according to that previously described, are only illustrative and not limitative of the present invention, since numerous changes of consideration in its details are possible without departing from the scope of the invention.

Therefore, the present invention should not be considered restricted except by the prior art demands and by the scope of the appended claims.

## Claims

1. A food supplement **characterized by** comprising, by weight: a) from 62 to 75% isomaltulose; b) from 12 to 21% milk serum protein concentrate; c) from 2 to 8.4% linseed; d) from 1 to 4.4% milk serum mineral complex; e) from 1.6 to 6.7% short chain fructooligosacharides; f) from 0.03 to 0.09% lactoferrin; and, g) from 0.002 to 0.008% vitamin B6.

2. A food supplement, according to claim 1, further comprising, by weight: a) from 65 to 71% isomaltulose; b) from 14 to 17% milk serum protein concentrate; c) from 4 to 4.5% linseed; d) from 2 to 2.5% milk serum mineral complex; e) from 3 to 4% short chain fructooligosacharides; f) from 0.05 to 0.07% lactoferrin; and, g) from 0.005 to 0.007% vitamin B6.

3. A food supplement, according to claim 1, wherein the milk serum protein concentrate has a protein content of about 80%.

4. A food supplement, according to claim 1, wherein the linseed has the following composition: about 41% fat, about 28% dietetic fiber, about 20% plant protein, about 7% humidity, and about 4% ashes.

5. A food supplement, according to claim 4, wherein the linseed has a two parts of omega-6 fatty acids to three parts of omega-3 fatty acids ratio.

6. A food supplement, according to claim 5, wherein the linseed employed is golden linseed.

7. A food supplement, according to claim 1, wherein the milk serum mineral complex has the following composition: about 24% calcium, about 12.50% phosphorous, about 1.50% magnesium, about 0.80% potassium, about 0.0080% zinc, and about 0.0004% copper.

8. A food supplement, according to claim 1, wherein short chain fructooligosacharides have the formula 1F-(1-β-fructofuranosyl)ₙ₋₁, where "n" may range between 2 and 4.

9. A food supplement, according to claim 1, wherein lactoferrin can be found in a holo lactoferrin or apolactoferrin form.

10. A food supplement, according to claim 1, wherein vitamin B6 can be found in a pyridoxine or pyridoxine hydrochloride form, preferably pyridoxine hydrochloride.

11. A food supplement, according to claim 1, comprising up to 13% by weight of one or more flavoring agents, preferably natural.

12. A food supplement, according to claim 1, comprising 2.5 to 5% by weight of a sweetener.

13. A food supplement, according to claim 12, wherein the sweetener is a stevia-based sweetener.

14. A food supplement, according to claim 1, having an osmolarity from 170 to 295 mOsm/L.

15. A food supplement, according to claim 1, being in a reconstitution powder, tablet, lozenge, pill, capsule, chewable dose unit, sachet, suspension, lollipop, candy, rubbery, or syrup form.

16. A food supplement, according to claim 15, being in a powder form to reconstitute in a liquid.

17. The use of the food supplement of claim 1 as support in the nutritional therapy on people suffering from Trisomy 21, Autism Spectrum Disorders and/or Attention Deficit Disorder with or without Hyperactivity.

18. The use of the food supplement, according to claim 17, wherein the route of administration is oral.

19. The use of the food supplement, according to claim 17, wherein said food supplement is administered twice a day.

20. The use of the food supplement, according to claim 19, wherein the first intake of the food supplement is performed before the first meal, and the second intake of the food supplement is performed in the afternoon.

21. The use of the food supplement, according to claim 20, wherein the first intake is performed before the first meal of the day, and the second intake is performed about 2 hours before night sleep.

22. The use of the food supplement, according to claim 17, wherein said food supplement may be used in combination with a diet where milk proteins, gluten, high glycemic index sugar, and red meat proteins have been replaced by casein-free milk, low glycemic index sugar, products with gluten-free flours, and white meat or plant proteins.

23. The use of the food supplement, according to claim 17, wherein said food supplement in people with Down Syndrome (Trisomy 21) allows to reduce the frequency of obstructive and central apneas; to record less events related to anxiety during sleep; to reduce reflux events; and, to increase the time in sleep stages III and IV.
